Europäisches Patentamt

European Patent Office

Office européen des brevets

(19)

(11) Publication number: **0 390 327**
**A2**

## (12) EUROPEAN PATENT APPLICATION

(21) Application number: **90301902.4**

(22) Date of filing: **22.02.90**

(51) Int. Cl.⁵: **C12N 5/00**

(30) Priority: **27.02.89 US 315887**

(43) Date of publication of application:
**03.10.90 Bulletin 90/40**

(84) Designated Contracting States:
**AT BE CH DE DK ES FR GB GR IT LI LU NL SE**

(71) Applicant: **ELI LILLY AND COMPANY**
**Lilly Corporate Center**
**Indianapolis Indiana 46285(US)**

(72) Inventor: **Prouty, Walter Francis**
**5520 East 79th Street**
**Indianapolis, Indiana 46250(US)**

(74) Representative: **Hudson, Christopher Mark et al**
**Erl Wood Manor**
**Windlesham Surrey GU20 6PH(GB)**

(54) **improved tissue culture method.**

(57) Arginyl-A-O insulin, a by-product of the biotechnological synthesis of human insulin from human proinsulin, is added to serum-free tissue culture media to stimulate the growth of mammalian cells which respond positively to insulin in tissue culture.

EP 0 390 327 A2

## IMPROVED TISSUE CULTURE METHOD

For some years, the art of growing mammalian cells in tissue culture has been at the heart of a number of fields of research in the biological sciences. Such cells are grown in tissue culture as sources of biochemical products, and for fundamental studies in biochemical and biotechnological processes. Mammalian cells growing in tissue culture are vital to substantially all of the recent advances in molecular biology.

Accordingly, research on tissue culture methods is presently of great importance, and numerous researchers are studying tissue culture conditions and the mechanisms which occur in tissue cultured cells.

The present invention belongs to the art of biochemistry, and particularly to the art of tissue culture.

The present invention provides a method of growing, in a serum-free tissue culture medium, mammalian cells which are suitable for growth in tissue culture and respond positively to the presence of insulin in the tissue culture medium, wherein the tissue culture medium contains from about 0.1 to about 10,000 nanograms of arginyl-A-O-insulin per milliliter.

The invention also provides a serum-free culture medium adapted to the growth of mammalian cells in tissue culture, comprising from about 0.1 to about 10,000 nanograms of arginyl-A-O-insulin per milliliter.

The present invention is characterized by the use of arginyl-A-O-insulin (AAO-insulin) as a growth-improving agent in tissue culture media for the growth of mammalian cells. AAO-insulin is a by-product in the process of preparing human insulin (HI) from human proinsulin. The process has been published in some detail and begins with human proinsulin covalently attached to a cleavable leader sequence which is synthesized by microorganisms genetically modified for that purpose. Frank, B. H., and Chance, R. E. (1983) Munch. Med. Wschr., 125 (Suppl. 1) 14-20. Frank, B. H., and Chance, R. E. (1985) Symposium "Quo Vadis?", Sanofi, May 29-30, Toulouse-Labege, France, pp. 138-146. Frank, B. H., Petee, J. M., Zimmerman, R. E., and Burck, P. J. (1981) in Peptides: Synthesis-Structure-Function. Proceedings of the Seventh American Peptide Symposium. D. H. Rick and E. Gross eds., Pierce Chemical Co., Rockford, Ill., pp. 729-738. The proinsulin is cleaved with trypsin and carboxypeptidase B, preferably in the presence of metal ions as taught by European Patent Publication 0264250 (U.S. Patent Application 06/917939).

As is now commonly known, human insulin consists of a 30-amino acid B chain linked to a 21-amino acid A chain through disulfide bridges which link the cysteines at position A-7 and B-7, and the cysteines at A-20 and B-19. Human proinsulin is a protein containing 86 amino acids, wherein the glycine at position A-1 of HI is linked to the threonine at position B-30 through an internal connecting peptide, a 35-amino acid chain. The connecting peptide is removed by cleavage in the process which converts human proinsulin to HI.

The cleavage process is not, of course, perfectly efficient, and some by-products result from imperfect cleavage. One such by-product is arginyl-A-O-insulin, which differs from HI in having one extra arginyl group, attached at position A-1, the amine terminus, of human insulin. That by-product may be separated from HI by cation exchange chromatography, as shown below in Preparation 1.

Interestingly, AAO-insulin is less potent than is HI in in vivo blood glucose lowering effects in rabbits. In the rabbitt assay, AAO-insulin was 75% as effective, mole for mole, as HI itself. However, AAO-insulin is at least as effective in stimulating the growth of mammalian cells in tissue culture as is insulin.

When biologists and biochemists first began to grow mammalian cells in tissue culture, it was conventional to grow them in tissue culture media which contained animal serum in concentrations in the 5-10% range. The serum provided valuable nutrients to the cells, and many important experiments have been carried out in such cultures. The desire for more precise experiments, however, have led to growth of tissue culture cells in defined media, wherein all the constituents are purified and accurately characterized chemical entities. One of the substances which should or must be added to defined media for the proper growth of many cells is insulin.

For example, the following cell types have been described in the literature as responding positively to the addition of insulin to tissue culture media.
Rat pituitary carcinoma
Human cervical carcinoma
Canine kidney carcinoma
Rat neuroblastoma
Mouse melanoma
Rat glioma
Human breast carcinoma
Rat ovary
Mouse embryonal carcinoma

Mouse testes
Human colon carcinoma
Balb/c mouse embryo
Rat myoblast
Human lung epidermoid carcinoma
Hamster kidney
Human fibroblast
Human epidermoid carcinoma
Rat thyroid
Mouse embryo
Mouse lymphoma
Sheep adipocyte

Clearly, insulin is a common necessary or desirable factor in media for the culture of mammalian cells. The use of the present invention is desirable in the culture of all mammalian cells which are stimulated by insulin in the culture medium. Thus, the nature or source of the cells to be used is not a limitation on the present invention.

Neither is the tissue culture medium a limiting factor. Serum-free tissue culture media have been extensively studied and published for 30 years or more. As a source of information about media for the convenience of the reader, the following references are suggested and are incorporated herein by reference.

Jayme and Blackman, Culture Media for Propagation of Mammalian Cells, Viruses and Other Biologicals, Advances in Biotechnological Processes 5, 1-30, A. R. Liss, Inc. (1985)

Ham and McKeehan, Methods Enzymol. 58, 44-93 (1979)

The Growth Requirements of Vertebrate Cells In Vitro, Waymouth, Ham and Chapple, Eds., Cambridge University Press (1981)

Methods for Serum-Free Culture of Cells of the Endocrine System, Barnes, Sirbasku and Sato, Eds., A. R. Liss, Inc. (1986)

In general, serum-free culture media for mammalian cells are made up of numerous ingredients including essential amino acids such as arginine, cysteine, histidine, methionine and the like; of non-essential amino acids, such as alanine, asparagine, glycine and the like; and amino acid derivatives such as glutathione, hydroxyproline, putrescine and the like. They also usually contain vitamins such as folic acid, biotin, nicotinamide, pantothenic acid, pyridoxine, riboflavin and vitamin B12. Carbohydrates such as glucose and pyruvate are used, as often are nucleic acid derivatives such as adenine, hypoxanthine and thymidine.

Such culture media contain a source of lipid such as choline and i-inositol, and inorganic ions including calcium, magnesium, sodium, phosphate and the like. Inorganic trace elements such as cobalt, iron, selenium and zinc are frequently included. Culture media are very often buffered to maintain the desired pH, often with bicarbonate ion or carbon dioxide gas. The Ham and McKeehan article cited above is a particularly good source of formulae of media which have been proved by successful use and are familiar in the art. An even more detailed description of tissue culture media can be found in the TCA Manual, published by the Tissue Culture Association, 12111 Park Lawn Drive, Rockville, MD. 20852.

As explained above, AAO-insulin is found as a by-product in the preparation of HI from human proinsulin, and is, accordingly, readily obtained for use in the present invention by mere isolation from the impure HI product stream. The following Preparation illustrates a chromatographic procedure useful for that isolation, and a crystallization step for the purification of the AAO-insulin.

Preparation 1

Chromatography

A mixture containing AAO-insulin and HI was dissolved in 32 ml of buffer containing 7M urea, 0.05M sodium acetate and 0.1M sodium chloride at pH 3.8. A 1 X 15 cm chromatography column was packed with Sulfopropyl TRISACRYL resin, made by Reactifs IBF, a division of Rhone-Poulenc, as 40-80 micron particles. The AAO-insulin solution was placed on the column, and was eluted with 9 column volumes of a gradient buffer of the same composition described above, except that the sodium chloride concentration rose to 0.2M. The flow rate was 1 ml/minute. The effluent from the column was monitored by absorption at 280 nm. It was found that the insulin came off first, and it had substantially all eluted in the first 70 ml after the start of the gradient elution. Then the AAO-insulin came off, and was collected in the effluent from 72-105 ml to obtain a fraction rich in AAO-insulin with very little contaminating HI.

Crystallization

A 840 ml portion of AAO-insulin product solution, containing 2.32 g of AAO-insulin, was adjusted to pH 8.0 with 10% sodium hydroxide. The solution was concentrated to 260 ml at 4°C using a 3000 molecular weight cutoff membrane. The concentrated solution was then diafiltered, with 280 ml of deionized water. The solution was diluted to a concentration of 2 g of protein per liter, and 16.2 ml of glacial acetic acid and 850 ml of deionized water were added to make the solution 0.25M in acetic acid. It was then warmed to 23°C and 0.93 ml of 20% aqueous zinc chloride was added. The pH was adjusted to 6.0 with concentrated ammonium hydroxide, and the mixture was stirred at 23°C for four hours. It was then cooled to 4°C, and the mixture was incubated at that temperature for 16 hours without stirring. Then the supernatant was decanted, and the thick crystalline slurry which remained was centrifuged. The crystals were washed twice with purified water and then twice with absolute ethanol, using 23 ml for each wash. The crystals were dried in vacuum at ambient temperature to obtain 2.11 g of essentially pure AAO-insulin.

## Example 1

AAO-insulin was used in the growth of Chinese hamster ovary cells in a serum-free medium which has been shown to be effective for growing such cells. The medium is composed of three parts of Dulbecco's modified Eagle's medium (DME) and one part (by volume) of F12 medium, with the addition of $10^{-8}$M selenium, 50μM ethanolamine, 20mM hydroxyethylpiperazine ethanesulfonic acid and 1 μg/ml of human transferrin. Amounts of AAO-insulin named in the table below were added to various portions of the medium. The cells were grown on 12-well Corning dishes, which were pre-treated with laminin, a protein which promotes cell attachment and spreading on the culture wells. Each condition was run in duplicate.

Each culture well full of medium was inoculated with 25,000 cells on day 0 of the experiment, and was counted with a Colter Counter on day 6. Four different lots of AAO-insulin were used in these experiments. The concentrations of AAO-insulin, which was added to the culture medium as a solution in 0.1N hydrochloric acid, are expressed in nanograms per milliliter in all cases.

In control experiments without the addition of insulin or AAO-insulin to the culture medium, the number of cells was from 10,000 to 200,000 cells per well. The same cells, grown in the same medium containing HI or beef insulin instead of AAO-insulin, grew as well as in the experiments described here.

| AAO-Insulin Concentration | Number of Cells (x1,000) | | | |
|---|---|---|---|---|
| ng/ml | Lot A | Lot B | Lot C | Lot D |
| 0.25 | 380 | 280 | 270 | 330 |
| 1 | 370 | 360 | 330 | 270 |
| 2.5 | 480 | 420 | 420 | 380 |
| 10 | 540 | 550 | 540 | 470 |
| 25 | 600 | 600 | 530 | 510 |
| 100 | 670 | 640 | 580 | 580 |

The above experiments show the effectiveness of AAO-insulin in stimulating the growth of cells which respond to insulin in tissue culture. Indeed, the average number of cells per well in these tests was increased to from 240,000 to 610,000, depending on concentration of AAO-insulin.

**Claims**

1. A serum-free tissue culture medium adapted for the growth of mammalian cells, comprising from about 0.1 to about 10,000 nanograms of arginyl-A-O insulin per milliliter.

2. A medium of Claim 1 wherein the concentration of arginyl-A-O insulin is from about 1 to about 1,000 nanograms per milliliter.

3. A medium of Claim 2 wherein the concentration of arginyl-A-O insulin is from about 1 to about 100 nanograms per milliliter.

4. A method of growing, in a tissue culture medium of Claim 1, mammalian cells which are suitable for growth in tissue culture and which respond positively to the presence of insulin in tissue culture medium.

5. A method of growing, in a tissue culture medium of Claim 2, mammalian cells which are suitable for growth in tissue culture and which respond positively to the presence of insulin in tissue culture medium.

6. A method of growing, in a tissue culture medium of Claim 3, mammalian cells which are suitable for growth in tissue culture and which respond positively to the presence of insulin in tissue culture medium.